# EUROPEAN PATENT APPLICATION

(11) **EP 3 620 192 A1**
(43) Date of publication of application: **11.03.2020**
(21) Application number: 18192891.2
(22) Date of filing: 06.09.2018
(51) Int. Cl.: A61M 3/02

(54) **EAR IRRIGATION DEVICE**

(71) Applicant: Sancare Health Co., Ltd, Taichung City (TW)
(72) Inventor: TSENG, Hung-Cheng, Taichung City (TW); TSENG, Jimmy, Taichung City (TW); TSENG, Howard, Taichung City (TW)
(74) Representative: Pallini Gervasi, Diego

(57) **Abstract**

An ear irrigation device has a tubular body (10) and an abutment member (20). The tubular body (10) has a central channel (12) and a curved irrigation orifice. The central channel (12) is defined as an internal cavity of the tubular body. The curved irrigation orifice (14) is defined radially in the tubular body (10) at a position adjacent to a front end (104) of the tubular body (10) and communicates with the central channel (12). The curved irrigation orifice (14) has a curvature larger than 90° and an inclined surface (142). The abutment member (20) is connected with the tubular body (10) by at least one connection rib (22) to define at least one curved drainage passage (24), and the area of one of the at least one curved drainage passage (24) is larger than half of the area defined by the abutment member (20).

## Description

### 1. Field of the Invention

The present invention relates to an ear irrigation device, and more particularly to an ear irrigation device that outputs fan-shaped irrigation spray, and allows fluid to drain out easily from the ear canal.

### 2. Description of Related Art

An ear irrigation device is applied to flush and wash away cerumen, pus or foreign body from an ear canal by pressured fluid. US Patent No. 6,706,023 discloses a conventional ear irrigation device comprising a tubular member and a flared member formed on one end of the tubular member. The tubular member has a bore and multiple orifices communicating with the bore. The flared member has multiple enclosed channels. In use, the said patented ear irrigation device is connected with a pressured fluid source via a tube, and the tubular member of the irrigation device is partially inserted into an ear canal. Consequently, pressured fluid is ejected into the ear canal via the bore and the orifices of the tubular member to flush the cerumen out from the ear canal.

However, the fluid ejected from the orifices of the said tubular member is a jet like, narrow, fluid beam; so the fluid will exert excessive pressure and force onto the inner surface of the ear canal and cause pain to the user. In addition, the area of the enclosed channels in the flared member of the said ear irrigation device, which is used for fluid drainage, is relatively small; so the stagnant fluid or cerumen in the ear canal does not easily flow out of the ear canal via the enclosed channels.

The present invention tends to provide an ear irrigation device to mitigate or obviate the aforementioned problems, and to further enhance the efficacy of cleansing the ear canal.

The main objective of the invention is to provide an ear irrigation device that can output fan-shaped irrigation spray for a larger cleansing area contact and drain fluid out from the ear canal easily.

The ear irrigation device has a tubular body and an abutment member. The tubular body has two ends, a central channel, and a curved irrigation orifice. The tubular body has a rear end and a front end. The central channel is defined as an internal cavity of the tubular body. The curved irrigation orifice is defined radially in the tubular body at a position adjacent to the front end and communicates with the central channel. The curved irrigation orifice has a curvature larger than 90° and an inclined surface formed on an inner surface being adjacent to the closed end of the tubular body. The abutment member is disposed around the tubular body at a position being adjacent to the open end of the tubular body and is connected with the tubular body by at least one connection rib to define at least one curved drainage passage between the tubular body and the abutment member, and the area of one of the at least one curved drainage passage is larger than half of the area defined by the abutment member.

Other objects, advantages and novel features of the invention will become more apparent from the following detailed description when taken in conjunction with the accompanying drawings.

### IN THE DRAWINGS

Fig. 1 is a perspective view of an ear irrigation device in accordance with the present invention;
Fig. 2 is another perspective view of the ear irrigation device in Fig. 1;
Fig. 3 is an front view of the ear irrigation device in Fig. 1;
Fig. 4 is a cross sectional side view of the ear irrigation device along the line 4-4 in Fig. 3;
Fig. 5 is a cross sectional front view of the ear irrigation device along the line 5-5 in Fig. 4;
Fig. 6 is an operational perspective view of the ear irrigation device in Fig. 1; and
Fig. 7 is an operational side view in partial section of the ear irrigation device in Fig. 1.

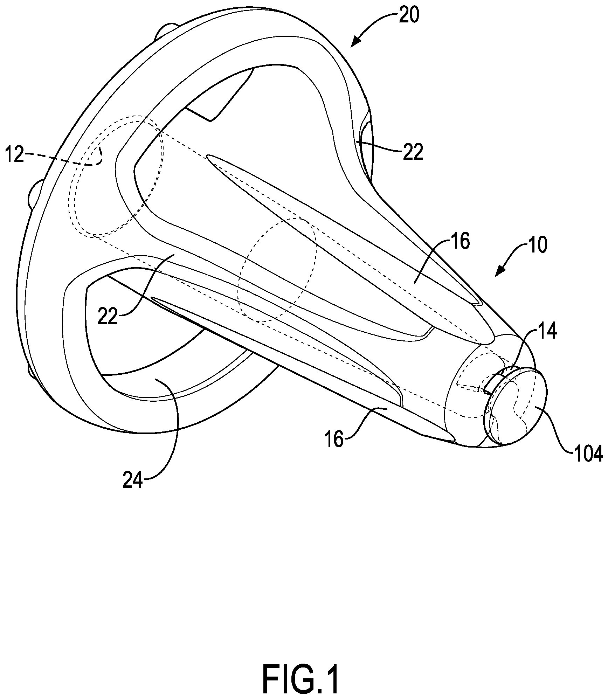

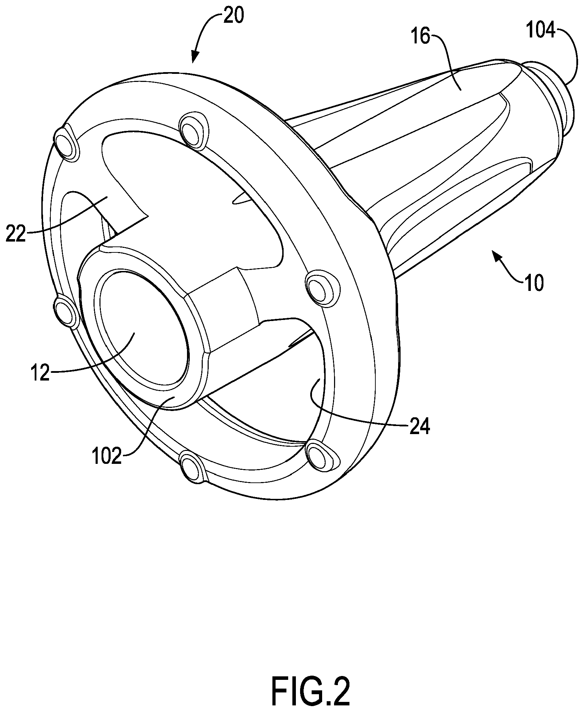

With reference to Figs. 1 to 4, an ear irrigation device in accordance with the present invention comprises a tubular body 10 and an abutment member 20.

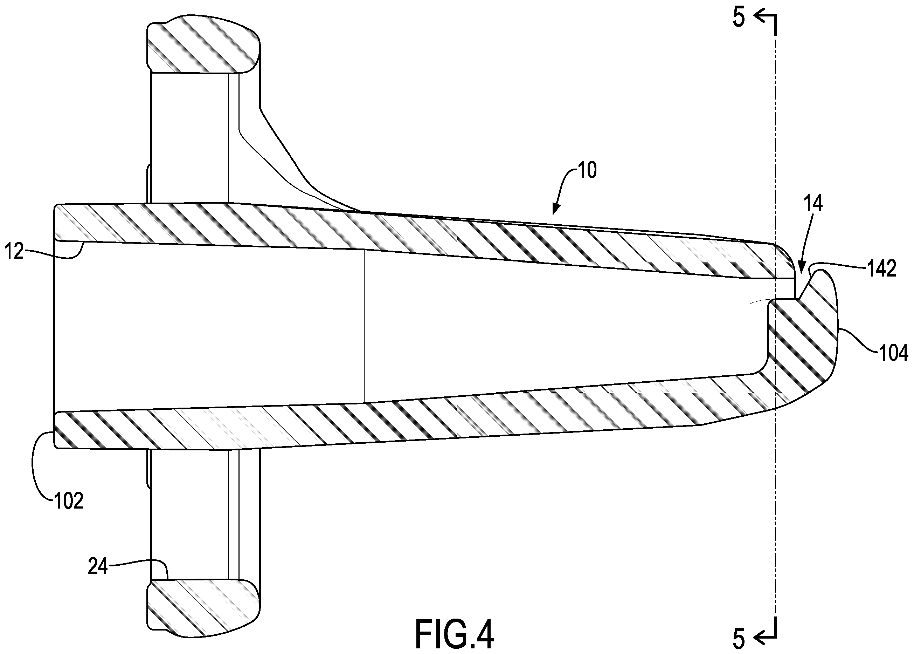

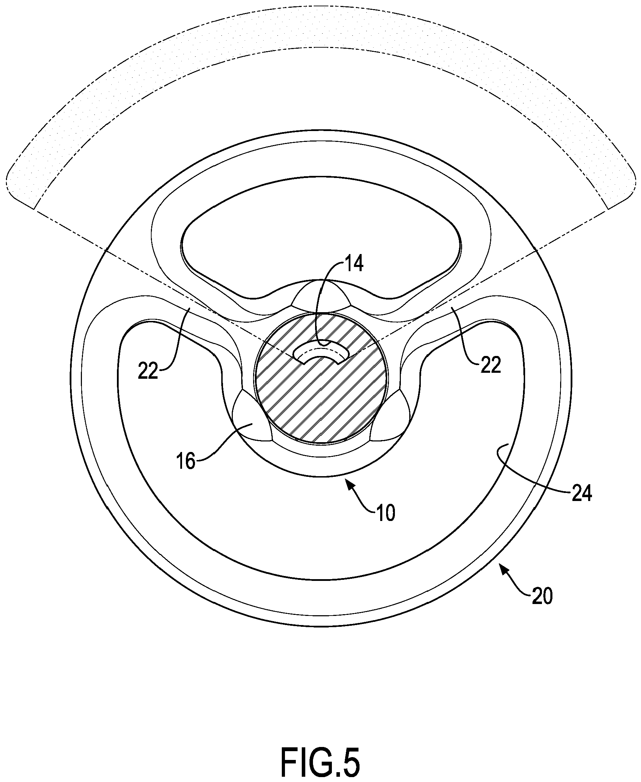

The tubular body 10 has two ends including a rear end 102and a front end 104, a central channel 12, and a curved irrigation orifice 14. The central channel 12 is defined in one of the rear end 102 of the tubular body 10 and is defined as an internal cavity of the tubular body 10. The curved irrigation orifice 14 is defined radially in the tubular body 10 at a position adjacent to the front end 104 and communicates with the central channel 12.With reference to Fig. 5, the arc length of the curved irrigation orifice 14 is preferably at least two times of the arc depth of the curved irrigation orifice 14. The curved irrigation orifice 14 has a curvature larger than 90°. With reference to Fig. 4, an inclined surface 142 formed on an inner surface being adjacent to the front end 104 of the tubular body 10 and has an angle between 40° to 80° relative to a vertical surface.

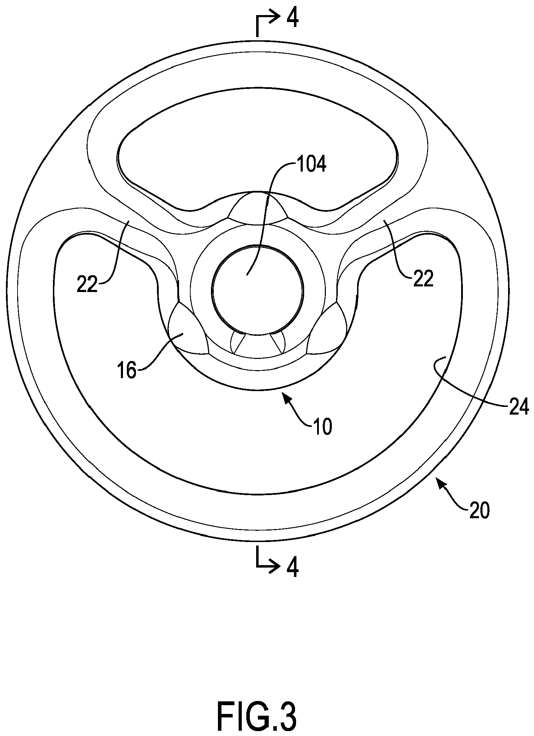

With reference to Figs. 3 and 5, the abutment member 20 may be annular, is disposed around the tubular body 10 at a position being adjacent to the rear end 102 of the tubular body 10, and is connected with the tubular body 10 by at least one connection rib 22. Accordingly, at least one curved drainage passage 24 is formed between the tubular body 10 and the abutment member 20, and one of the at least one curved drainage passage 24 has an area larger than half of the entire area within the outer perimeter of the abutment member 20 excluding that of the tubular body 10; where said areas are areas viewed from top or rear, perpendicular to the tubular body 10. With reference to Fig. 5, the larger curved drainage passage 24 is positioned on the opposite side of the curved irrigation orifice 14.

In addition, at least one guiding groove 16 is defined longitudinally in an outer surface of the tubular body 10 and communicates with the drainage passages 24.

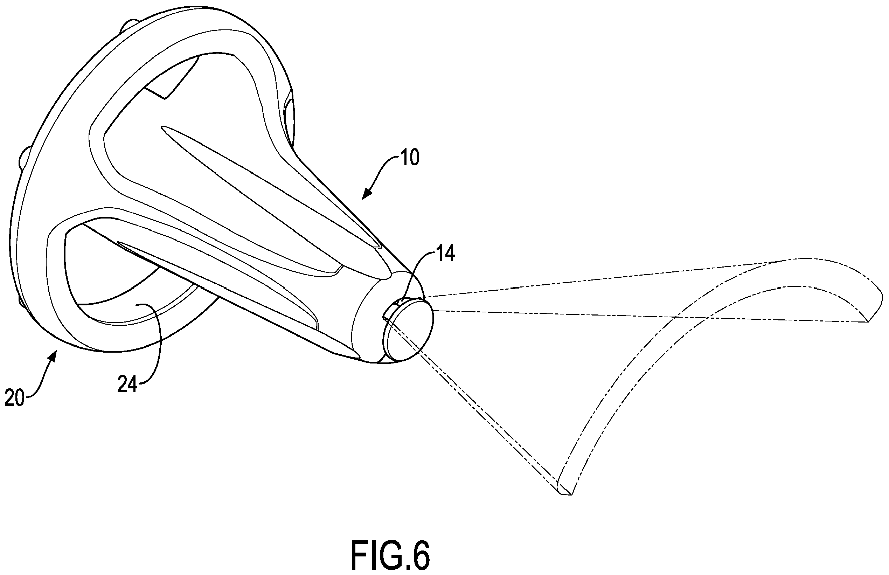

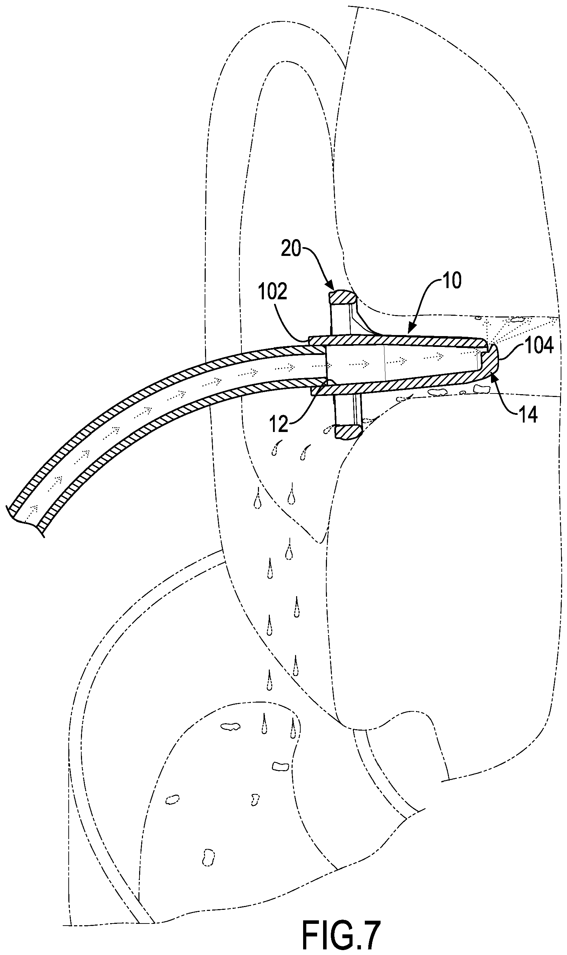

In use, with reference to Figs. 4 to 7, the rear end 102 of the tubular body 10 is connected with a pressured fluid source by a tubing, an irrigation tip, or a syringe, and the front end 104 of the tubular body 10 is inserted into an ear canal and the abutment member 20 abuts the orifice of the ear canal. Accordingly, pressured fluid can be led into the central channel 12 in the tubular body 10 and is ejected out from the curved irrigation orifice 14 in a curved fan-shaped form. Once the fluid is ejected from the curved irrigation orifice 14, the fluid will bump against the inclined surface 142, to divert the fluid at an angle from the axis defined by the central channel 12. The bump of the fluid and the curved fan-shaped irrigation together can effectively reduced the force of the ejected fluid applied to the ear canal and prevent the user from feeling pain. The fan-shaped fluid beam also provides a larger cleansing area. In addition, because a large drainage passage 24 has a curvature larger than 180°, the area of the curved drainage passage 24 formed between the tubular body 10 and the abutment member 20 is relatively large. Therefore, the fluid, cerumen, pus, or foreign body in the ear canal can be easily drained from the ear canal via the large curved drainage passage 24.

## Claims

1. An ear irrigation device, **characterized in that** the ear irrigation device comprises:
a tubular body (10) comprising
two ends (102, 104) including an rear end (102) and a front end (104);
a central channel (12) defined as an internal cavity of the tubular body; and
a curved irrigation orifice (14) defined radially in the tubular body (10) at a position adjacent to the frontend (104), communicating with the central channel (12), and having a curvature larger than 90° and an inclined surface (142) formed on an inner surface being adjacent to the front end (104) of the tubular body (10); and
an abutment member (20) disposed around the tubular body (10) at a position being adjacent to the rear end (102) of the tubular body (10) and connected with the tubular body (10) by at least one connection rib (22) to define at least one curved drainage passages (24) between the tubular body (10) and the abutment member (20).

2. The ear irrigation device as claimed in claim 1, wherein the curved irrigation orifice (14) is defined radially in the tubular body (10) has an arc length that is at least two times larger than the arc depth of the curved irrigation orifice (14) to irrigate fluid in a curved fan-shaped form.

3. The ear irrigation device as claimed in claim 2, wherein the inclined surface (142) of the curved irrigation orifice (14) has an angle between 40° to 80° relative to a vertical surface.

4. The ear irrigation device as claimed in claim 3, wherein one of the at least one curved drainage passage (24) has an area larger than half of the area defined by the abutment member (20).

5. The ear irrigation device as claimed in claim 4, wherein the curved drainage passage (24) having the area larger than the area defined by the abutment member (20) is positioned on an opposite side relative to the curved irrigation orifice (14).
